(19) Europäisches Patentamt
European Patent Office
Office européen des brevets

(11) Publication number: 0 231 958 B1

# (12) EUROPEAN PATENT SPECIFICATION

(45) Date of publication of the patent specification: 27.12.89

(51) Int. Cl.⁴: C07J 7/00, A61K 31/57 // C07J31/00, C07J71/00

(21) Application number: 87200009.6

(22) Date of filing: 06.01.87

## (54) Novel pregnane derivatives, processes for their preparaton and pharmaceutical compositions containing same.

(30) Priority: 15.01.86 GB 8600879

(43) Date of publication of application: 12.08.87 Bulletin 87/33

(45) Publication of the grant of the patent: 27.12.89 Bulletin 89/52

(84) Designated Contracting States: AT BE CH DE ES FR GB GR IT LI NL SE

(56) References cited:
US-A- 3 162 630
US-A- 3 483 236

CHEMICAL ABSTRACTS, vol. 78, no. 8, 26th February 1973, page 278, abstract no. 47795p, Columbus, Ohio, US; & NL-A-71 05 591 (AKZO N.V.) 26-10-1972

The file contains technical information submitted after the application was filed and not included in this specification

(73) Proprietor: AKZO N.V., Velperweg 76, NL-6824 BM Arnhem(NL)

(72) Inventor: Logan, Robert Thomas, Leven 1 Staik Hill Lanark ML 11 7 PW, Lanarkshire Scotland(GB)
Inventor: Woods, Gilbert Frederick, Llwyn Onn Anglesey Gwynedd, Llanfaes Beaumaris Wales(GB)

(74) Representative: Hermans, Franciscus G.M. et al, Patent Department AKZO N.V. Pharma Division P.O. Box 20, NL-5340 BH Oss(NL)

EP 0 231 958 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel pregnane derivatives substituted in 21-position by F , Cl or Br, to processes for their preparation and to pharmaceutical compositions containing one or more of said derivatives as active constituent.

More particularly, the invention relates to novel pregnane derivatives of the formula:

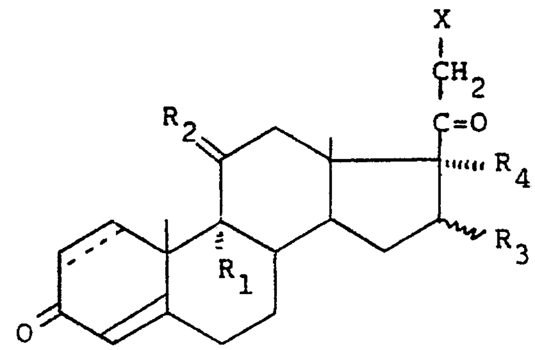

wherein

X = F, Cl or Br, preferably Cl:

$R_1$ = F or Cl, preferable F;

$R_2$ = H(βOH) or O, preferably H(βOH);

$R_3$ = alkyl(1-4 C), preferably $CH_3$ which is preferably in α-position;

$R_4$ = alkyl(1-4 C), preferably $CH_3$; and

the dotted line indicates the optional presence of a double bond in 1,2-position, said double bond preferably being present.

Related pregnane derivatives are known, e.g. compounds substituted with halogen at C9 and C21 (US-A 3 162 630) and compounds substituted with alkyl at C16 and C17 (US-A 3 483 236). Those compounds lack, however, the immunomodulating properties of the compounds according to this invention.

The compounds of the present invention possess very interesting immunomodulating properties. They exhibit a higher affinity for the corticosteroid leucocyte receptors and have reduced hormonal (glucocorticoid) effects compared with known glucocorticoids such as dexamethasone. In vitro and in vivo tests have shown that the novel compounds of the present invention, particularly the 21-chloro compounds, stimulate the humoral immune response over a wide range of relatively low doses and suppress the cell mediated immune response at relatively high doses. At low doses the novel compounds cause a functionel maturation of the immune system in response to antigen stimulation.

The compounds according to the invention are therefore useful for treating a wide range of diseases involving a disorder of the immune system, e.g. cancer, auto-immune diseases, and also for preventing the rejection of transplants.

The compounds within the purview of this invention can be prepared by methods obvious to those skilled in the art. They may be prepared e.g. by starting from the corresponding 21-hydroxy steroid and converting the 21-hydroxy group into the 21-sulphonate thereof, e.g. into the 21-mesylate or the 21-tosylate, after which the 21-sulphonate is reacted with an alkalimetal halide, e.g. lithium chloride or potassium fluoride, to obtain the desired 21-halo steroid.

The conversion of the 21-hydroxy steroid into the 21-sulphonate is carried out by reaction with the appropriate sulphonic acid halide, such as methanesulphonyl chloride or toluenesulphonyl chloride, at low temperature (0-20 °C) in the presence of a base, preferably an organic base such as pyridine, that can also serve as the solvent. The reaction of the 21-sulphonate with the alkalimetal halide is carried out in a suitable solvent, such as dimethylformamide, dimethylacetamide or ethyleneglycol, at elevated temperature, conveniently under reflux.

It is also possible to convert the 21-hydroxy steroid into the 21-halo steroid directly by reacting the 21-hydroxy steroid with a sulphonyl halide, such as methanesulphonyl chloride, in dimethylformamide at elevated temperature, i.e. in the range of 50 to 70 °C.

Another method for preparing the compounds according to the invention is to start from the corresponding $\Delta^{9(11)}$ compound and introducing into 9,11-position the required substituents by methods known in the art, such as converting the $\Delta^{9(11)}$ double bond into the 9α-bromo-1β-hydroxy compound or an 11β-ester thereof, e.g. by reaction with N-bromo-succinimide in dimethylformamide in the presence of perchloric acid, and transforming the 9α-bromo-11β-hydroxy compound or the 11β-ester thereof under basic conditions into the corresponding 9β,11β-epoxide, which is subsequently opened with a halogen acid to give the desired 9α-halo-11β-hydroxy derivative. Reacting a $\Delta^{9(11)}$ compound with N-chloro-succinimide gives directly the 9α-chloro-11β-hydroxy compound.

Starting materials for the above reactions are described in literature, e.g. in U.S. Patent Specification 3 520 908 or can readily be prepared from the 16α,17α-dialkyl compounds described in said U.S. Specification, e.g. 21-hydroxy-$\Delta^{9(11)}$ compounds can be converted into the corresponding 21-halo-$\Delta^{9(11)}$ compounds by the method described hereinbefore or 11,21-dihydroxy compounds can be converted into the corresponding 21-halo-$\Delta^{9(11)}$ compounds in a one-step synthesis using an organic sulphonylchloride, such as methane sulphonylchloride, in pyridine.
The compounds according to the invention can also be prepared starting from the corresponding 21-iodo compound (see e.g. U.K. Patent Specification 1 458 517) and reacting this compound with an alkalimetal chloride, bromide, or fluoride in a suitable solvent such as N-methyl-pyrrolidone.

In compounds according to the invention containing a $\Delta^4$-3-oxo group an additional double bond in position $C_1$-$C_2$ may be introduced by known chemical means such as by reaction with a suitable quinone derivative, e.g. dichlorodicyanobenzoquinone, or with seleniumdioxide or a derivative thereof such as diphenyldiseleninic anhydride.
A double bond in position $C_1$-$C_2$ may also be introduced microbiologically by incubation with a 1,2-dehydrogenating micro-organism, for example <u>Corynebacterium simplex</u>, <u>Bacillus sphaericus</u> or <u>Bacillus subtillis.</u>

A $\Delta^{1,4}$-3-oxo compound can be converted into a $\Delta^4$-3-oxo compound by $\Delta^1$-hydrogenation, e.g. by catalytic hydrogenation in the presence of the homogenous catalyst tris-triphenylphosphine rhodium (I) chloride.

An 11-hydroxy group may, if required, be oxidised to an 11-keto group, e.g. with chromic acid.

If an intermediate contains already an 11-hydroxy group, but not yet the 9α-fluoro or -chloro group, such intermediate is dehydrated in 9,11-position and the $\Delta^{9(11)}$ compound thus obtained is converted into a 9α-halo-11β-hydroxy compound by the method described hereinbefore.

The compounds of the present invention can be administered enterally or parenterally in the usual administration forms, i.e. pharmaceutical compositions, for which purpose they are mixed with one or more pharmaceutically acceptable non-toxic carriers and/or the usual excipients.
The pharmaceutical compositions include tablets, pills, coated tablets and suppositories for enteral administration and solutions, suspensions and emulsions for parenteral administration (injection).

Other methods of administration include sublingual administration, nasal sprays, inhalation and topical administration in the form of ointments, creams, lotions of sprays.

The invention is further illustrated by the following Examples.

<u>Example I</u>

a) <u>9α-Fluoro-11β,21-dihydroxy-16α, 17α-dimethylpregna-1,4-diene-3,20-dione 21-methanesulphonate</u>.

To a stirred suspension of 9α-fluoro-11β,21-dihydroxy-16α,17α-dimethylpregna-1,4-diene-3,20-dione (2.5 g) in pyridine (12.5 ml) was added methanesulphonyl chloride (1.25 ml) while the temperature was maintained between 2 °C and 10 °C. The mixture was stirred for a further 30 min. at below 10 °C. Water (50 ml) was added and while stirring was continued, the precipitated gum solidified. The solid was filtered off, washed with water and dried <u>in vacuo</u>. Recrystallisation from acetone-ether gave the title methanesulphonate (2.77 g), m.p. 229-231 °C (dec), $[\alpha]_D^{20}$(pyridine) +102°.

b) <u>21-Chloro-9α-fluoro-11β-hydroxy-16α, 17α-dimethylpregna-1,4-diene-3,20-dione</u>.

Lithium chloride (1.5 g) was added to a solution of 9α-fluoro-11β,21-dihydroxy-16α,17α-dimethylpregna-1,4-diene-3,20-dione 21-methanesulphonate (3 g) in dimethylformamide (30 ml) and the mixture was heated under reflux for 20 min. After cooling, the mixture was poured into water and the precipitated solid was filtered off, washed with water and dried <u>in vacuo</u>. Chromatography on silica and recrystallisation of the mid-fractions eluted from methylene chloride-ether (1:1) gave the title chlorosteroid (1.75 g), m.p. 252-254 °C; $[\alpha]_D^{20}$ ($CHCl_3$) +64°.

<u>Example II</u>

<u>21-Chloro-9α-fluoro-16α,17α-dimethyl-pregna-1,4-diene3,11,20-trione</u>

Jones reagent (2.07M; 2.9 ml) was added dropwise to a solution of 21-chloro-9α-fluoro-11β-hydroxy16α,17α-dimethyl-pregna-1,4-diene-3,20-dione (960 mg) in acetone (25 ml) at 0 °C. The reaction mixture was poured into water at 0 °C. The precipitated solid was filtered off and dissolved in methylene chloride. The solution was washed with sodium carbonate at 0 °C and then with water and dried over sodium sulphate. Removal of the solvent under reduced pressure gave a solid (954 mg) which recrystallised from acetone to give the title compound (840 mg) as colourless needles, m.p. 238-240 °C.

Example III

9α,21-Difluoro-11β-hydroxy-16α,17α-dimethyl-pregna-1,4-diene-3,20-dione

Anhydrous potassium fluoride (4.16 g) was added to a suspension of 9α-fluoro-11β,21-dihydroxy-16α,17α-dimethylpregna-1,4-diene-3,20-dione 21-methanesulphonate (2.08 g) in ethyleneglycol (45 ml) and the mixture heated under nitrogen for 1 h. at 165 °C. The mixture was poured into water at 0 °C and the precipitated solid was filtered off. Chromatography and recrystallisation from chloroformmethanol afforded the title compound, m.p. 324-326 °C (decomp.).

Example IV

9α,21-Difluoro-16α,17α-dimethylpregna-1,4-diene-3,11,20-trione

Jones reagent (2.7M) was added to a stirred suspension of 9α,21-difluoro-11β-hydroxy-16α,17α-dimethyl-pregna-1,4-diene-3,20-dione (400 mg) until oxidation of the 11-hydroxy function was complete. The mixture was poured into water at 0 °C, and the precipitated solid was filtered off and dissolved in methylene chloride. The solution was dried over sodium sulphate and the solvent removed in vacuo. Recrystallisation from ether gave the title compound, m.p. 223-224 °C.

Example V

In a similar way as described in Example I or III the following compounds were prepared, starting from the corresponding 21-hydroxy compounds:
21-chloro-9α-fluoro-11β-hydroxy-16α,17α-dimethyl-pregn-4-ene-3,20-dione;
21-chloro-16α-ethyl-9α-fluoro-11β-hydroxy-17α-methyl-pregna-1,4-diene-3,20-dione;
21-chloro-17α-ethyl-9α-fluoro-11β-hydroxy-16α-methyl-pregn-4-ene-3,20-dione;
16α-ethyl-9α,21-difluoro-11β--hydroxy-17α-methyl-pregna-1,4-diene-3,20-dione;
21-bromo-16α-ethyl-9α-fluoro-11β-hydroxy-17α-methyl-pregn-4-ene-3,20-dione;
21-bromo-9α-fluoro-11β--hydroxy-16α,17α-dimethyl-pregna-1,4-diene-3,20-dione;
9α,21-dichloro-11β-hydroxy-16α,17α-dimethyl-pregna-1,4-diene-3,20-dione:
which are then converted into the corresponding 11-ketones in a similar way as described in Example II or IV.

Example VI

21-Chloro-9α-fluoro-11β-hydroxy-16α,17α-dimethyl-pregna-1,4-diene-3,20-dione

Methanesulphonyl chloride (0.5 ml) was added to a solution of 11β,21-dihydroxy-9α-fluoro-16α,17α-dimethyl-pregna-1,4-diene-3,20-dione in dimethylformamide (20 ml) and the mixture was heated at 60 °C for 1 hour. Then a second amount of methanesulphonyl chloride (0.5 ml) was added and the mixture was heated at 60 °C for 3 hours, then poured into water at 0 °C, the precipitate filtered off, washed and recrystallized from acetone-ether to give the title compound (0.5 g), m.p. 251-254 °C.

Example VII

21-Chloro-9α-fluoro-11β-hydroxy-16α,17α-dimethylpregna-1,4-diene-3,20-dione.

Lithium chloride (1.26 g) was added to a solution of 9α-fluoro-11β-hydroxy-21-iodo-16α,17α-dimethyl-pregna-1,4-diene-3,20-dione (9.5 g) in N-methyl-2-pyrrolidinone (190 ml) and the stirred mixture was heated at 55°C for 1 h in an atmosphere of nitrogen. The mixture was cooled and then poured into water at 0°C. The precipitated solid was filtered off, washed with water and dissolved in methylene chloride. The solution was washed with water, dried over sodium sulphate and the solvent was removed under reduced pressure. The resultant colourless solid (7.8 g) was recrystallised from methylene chloride-ether to give 21-chloro-9α-fluoro-11β-hydroxy-16α,17α-dimethylpregna-1,4-diene-3,20-dione (5-6 g), m.p. 252-254°C; $[\alpha]_D^{20}$ ($CHCl_3$) + 64°.

Example VIII

a) 21-Chloro-11β-hydroxy-16α,17α-dimethylpregna-1,4-diene-3,20-dione.

Lithium chloride (1.26 g) was added to a solution of 21-iodo-11β-hydroxy16α,17α-dimethylpregna-1,4-diene-3,20-dione (9.5 g) in N-methyl-2-pyrrolidinone (190 ml) and the stirred mixture was heated at 55°C for 1 h in an atmosphere of nitrogen. The mixture was cooled and then poured into water at 0°C. The precipitated solid was filtered off, washed with water and dissolved in methylene chloride. The solution was washed with water, dried over sodium sulphate and the solvent was removed under reduced pressure. The resultant colourless solid (7.8 g) was recrystallised from methylene chloride-ether to give 21-chloro-11β-hydroxy-16α, 17α-dimethylpregna-1,4-diene-3,20-dione (5-6 g), m.p. 260-265°C; $[\alpha]_D^{20}$ ($CHCl_3$) + 63.9°.

b) 21-Chloro-16α,17α-dimethylpregna-1,4,9(11)-triene-3,20-dione.

To a stirred mixture of 21-chloro-11β-hydroxy-16α,17α-dimethylpregna-1,4-diene-3,20-dione (7.8 g) in dimethylformamide (78 ml) and collidine (23 ml) at 10°C was added dropwise over 3-4 min a solution (3.9 ml) of methanesulphonyl chloride containing sulphur dioxide (5%). The mixture was then stirred for 1.75 h at ambient temperature and sulphuric acid (5N; 700 ml) at 0°C was added. The precipitated solid was filtered off, washed with water and then dissolved in methylene chloride. The solution was washed with water until free of acid and dried over sodium sulphate. Removal of the solvent under reduced pressure gave a solid (6.7 g ) which was dissolved in toluene and chromatographed on a column of silica. The fractions eluted with toluene-ethyl acetate (19:1) were evaporated to dryness to give a solid which was recrystallised from acetone-hexane to give 21-chloro- 16α,17α-dimethylpregna-1,4,9(11)-triene-3,20-dione (4.6 g), m.p. 106-162°C;$[a]_D^{20}$ ($CHCl_3$ - 1.9°.

c) 9α-Bromo-21-chloro-11β-hydroxy-16α,17α-dimethylpregna-1,4-diene-3,20-dione 11-formate.

A stirred solution of 21-chloro-16α,17α-dimethylpregna-1,49(11)-triene-3,20-dione (78 g) in dimethylformamide (816 ml) was treated dropwise at 5°C with perchloric acid (70%; 19.6 ml) keeping the temperature below 10°C. N-Bromosuccinimide (57.2 g) was added over 10 min and the mixture was stirred for a further 1.25 h at 8-10°C. Sodium bisulphite (7 g) in water (40 ml) was added and the mixture was poured into water (8 l) at 0°C. The precipitated solid was filtered off, and dissolved in methylene chloride. The solution was washed with water, dried over sodium sulphate and the solvent was removed under reduced pressure to give 9α-bromo-21-chloro-11β-hydroxy-16α,17α-dimethylpregna-1,4-diene-3,20-dione 11-formate (99.6 g).

d) 21-Chloro-16α,17α-dimethyl-9β,11β-oxidopregna-1,4-diene-2,30-dione.

To a stirred solution of 9α-bromo-21-chloro-11β-hydroxy-16α,17α-dimethylpregna-1,4-diene-3,20-dione 11-formate (99 g) in tetrahydrofuran (990 ml) at 6°C was added a solution of sodium methoxide (12.95 g) in methanol (990 ml). The mixture was stirred at 6-10°C for 2 h and the solution was then adjusted to pH6 with acetic acid. The mixture was concentrated to one half of the volume and then poured into water (4 l) at 0°C. Isolation of the product through ether and then methylene chloride gave a yellow solid (68.4 g) Two recrystallisations from methylene chloride-ether gave 21-chloro-16α,17α-dimethyl-9β,11β-oxidopregna-1,4-diene-3,20-dione (657 mg), m.p. 201-202°C; $[\alpha]_D^{20}$ ($CHCl_3$) + 18.6°.

e) 21-Chloro-9α-fluoro-11β-hydroxy-16α,17α-dimethylpregna-1,4-diene-3,20-dione.

To a stirred suspension of 21-chloro-16α,17α-dimethyl-9β,11β-oxidopregna-1,4-diene-3,20-dione (1.0 g) in diglyme (9.5 ml) at 0°C was added a solution of hydrogen fluoride in diglyme (12M; 0.63 ml) cooled to 0°C, immediately followed by boron trifluoride etherate in ether (45% $BF_3$; 0.95 ml) cooled to 0°C. The mixture was stirred for a further 2 h at ambient temperature and then poured into water (50 ml) containing sodium acetate (2.84 g). The precipated solid was filtered off, washed with water and dissolved in methylene chloride. The solution was washed with water, dried over sodium sulphate and the solvent was removed under reduced presure to give a solid (1.1 g). Recrystallisation from methylene chloride-ether gave 21-chloro-9α-fluoro-11β-hydroxy-16α, 17α-dimethylpregna-1,4-diene-3,20-dione (708 mg), m.p. 252-254°C; $[\alpha]_D^{20}$($CHCl_3$) + 64°.

Example IX

9α,21-Dichloro-11β-hydroxy-16α,17α-dimethylpregna-1,4-diene-3,20-dione.

To a mixture of 21-chloro-16α,17α-dimethylpregna-1,4,9(11)-triene-3,20-dione (0.5 g), dioxan (9.5 ml) and water (0.7 ml) at 20°C was added portionwise N-chlorosuccinimide (0.5 g) and then a solution of perchloric acid (70%; 0.05 ml) in water (0.8 ml). The mixture was stirred at room temperature for 70 h. A solution of sodium acetate (200 mg) and sodium bisulphite (125 mg) in water (1.5 ml) was added and the mixture was poured into water (100 ml) at 0°C. The precipitated solid was filtered off and dissolved in methylene chloride. The solution was washed with water, dried over sodium sulphate and the solvent was removed under reduced pressure. Trituration with ether and recrystallisation from acetone afforded 9α,21-dichloro-11β-hydroxy-16α,17α-dimethylpregna-1,4-diene-3,20-dione, m.p. 235-241°C (decomp.).

Example X

21-Bromo-9α-fluoro-11β-hydroxy-16α,17α-dimethylpregna-1,4-diene-3,20-dione.

A stirred suspension of 9α-fluoro-11β,21-dihydroxy-16α,17α-dimethylpregna-1,4-diene-3,20-dione 21-methanesulphonate (454 mg) and sodium bromide (1.14 g) in acetone (21.4 ml) was heated under reflux for 28 h and the mixture was then poured into water (200 ml). The precipitated solid was filtered off, washed with water and then dried under reduced pressure at 70°C. The resultant colourless solid was dissolved in methylene chloride and chromatographed on a column of silica. The fractions eluted with ether through to methylene chloride-ether (1:1) gave a white solid (380 mg) which was recrystallised from methylene chloride-ether to give 21-bromo-9α-fluoro-11β-hydroxy-16α,17α-dimethylpregna-1,4-diene-3,20-dione (290 mg), m.p. 195-200°C (decomp.); $[\alpha]_D^{20}$ + 71.0° ($CHCl_3$).

Example XI

a) 21-Chloro-16α,17α-dimethylpregna-1,4,9(11)-diene-3,20-dione.

A solution of 21-hydroxy-16α,17α-dimethylpregna-1,4,9(11)-triene-3,20-dione (200 mg), methanesulphonyl chloride (0.1 ml) and dimethylformamide (2 ml) was heated at 60°C for 1.5 h and the mixture was then poured into water (25 ml). The precipitated solid was filtered off and dissolved in methylene chloride. The solution was washed with water, dried over sodium sulphate and the solvent was removed under reduced pressure. The resultant gum (240 mg) was crystallised from acetone-petroleum ether (60-80) to give 21-chloro-16α,17α-dimethylpregna-1,4,9(11)-triene-3,2-dione (135 mg), m.p. 160-162°C; $[\alpha]_D^{20}$ ($CHCl_3$) - 1.9°.

b) 21-Chloro-9α-fluoro-11β-hydroxy-16α,17α-dimethylpregna-1,4-diene-3,20-dione.

In a similar way as described in Example VIII, c) - e), the product of Example XI a) was converted into the title compound, m.p. 252 - 254°C; $[\alpha]_D^{20}$ ($CHCl_3$) + 64°.

**Claims**

1. Novel pregnane derivatives having the formula:

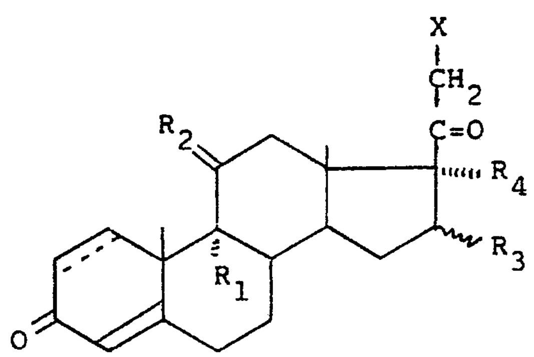

wherein
X = F, Cl or Br;
$R_1$ = F or Cl;
$R_2$ = H(βOH) or O;
$R_3$ = alkyl(1-4 C);
$R_4$ = alkyl(1-4 C); and
the dotted line indicates the optional presence of a double bond in 1,2-position.

2. Compound according to claim 1, characterized in that X = Cl.
3. Compound according to claim 1 or 2, characterized in that $R_1$ = F.
4. Compound according to claims 1-3, characterized in that $R_2$ = H(βOH).
5. Compound according to claims 1-4, characterized in that $R_3$ = $CH_3$.
6. Compound according to claims 1-5, characterized in that $R_3$ = α-$CH_3$.
7. Compound according to claims 1-6, characterized in that $R_4$ = $CH_3$.
8. Compound according to claims 1-7, characterized in that the double bond in 1,2-position is present.
9. Process for preparing a pregnane derivative having the formula

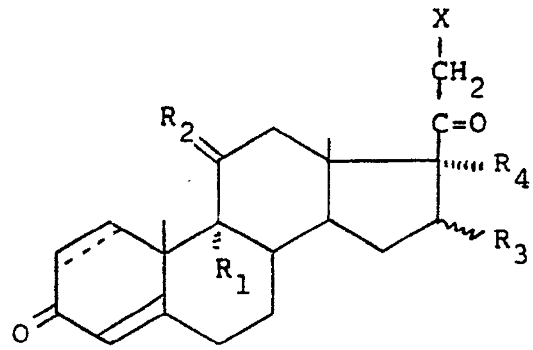

wherein
X = F, Cl or Br;
$R_1$ = F or Cl;
$R_2$ = H(βOH) or O;
$R_3$ = alkyl(1-4 C);
$R_4$ = alkyl(1-4 C) and
the dotted line indicates the optional presence of a double bond in 1,2-position by converting the corresponding 21-OH compound into the corresponding 21-halo compound, or the corresponding 9β,11β-epoxide into the corresponding 9α-halo, 11β-OH compound, or the corresponding $\Delta^{9(11)}$- the corresponding 21-I compound into the corresponding 21-Br, 21-Cl or 21-F compound and by, optionally, providing the compound obtained according to the above methods with a double bond in position $C_1$-$C_2$ or hydrogenating its double bond in position $C_1$-$C_2$ and/or oxidizing its 11β-OH-group.

**Patentansprüche**

1. Neue Pregnan-Derivate mit der Formel:

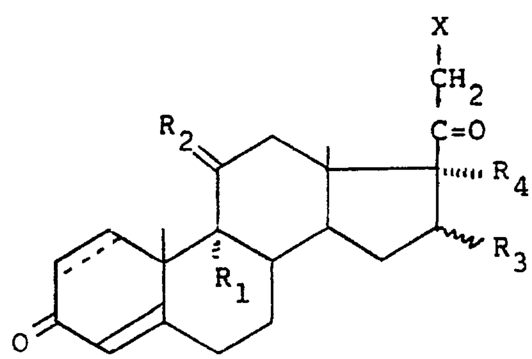

worin
X = F, Cl oder Br;
$R_1$ = F oder Cl;
$R_2$ = H(β-OH) oder O;
$R_3$ = $C_{1-4}$-Alkyl;
$R_4$ = $C_{1-4}$-Alkyl bedeuten und die gestrichelte Linie eine gegebenenfalls vorhandene Doppelbindungen in der 1,2-Stellung angibt.

2. Verbindung nach Anspruch 1, dadurch gekennzeichnet, daß X = Cl bedeutet.
3. Verbindung nach Anspruch 1 oder 2, dadurch gekennzeichnet, daß $R_1$ = F bedeutet.
4. Verbindung nach Ansprüchen 1–3, dadurch gekennzeichnet, daß $R_2$ = H(β-OH) bedeutet.
5. Verbindung nach Ansprüchen 1–4, dadurch gekennzeichnet, daß $R_3$ = $CH_3$ bedeutet.
6. Verbindung nach Ansprüchen 1–5, dadurch gekennzeichnet, daß $R_3$ = α-$CH_3$ bedeutet.
7. Verbindung nach Ansprüchen 1–6, dadurch gekennzeichnet, daß $R_4$ = $CH_3$ bedeutet.
8. Verbindung nach Ansprüchen 1–7, dadurch gekennzeichnet, daß sich die Doppelbindung in der 1,2-Stellung befindet.
9. Verfahren zur Herstellung eines Pregnan-Derivats mit der Formel:

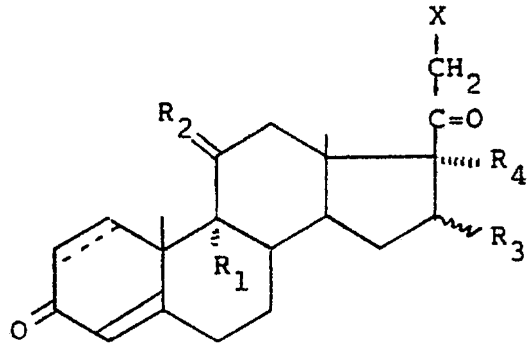

worin
X = F, Cl oder Br;
$R_1$ = F oder Cl;
$R_2$ = H(β-OH) oder O;
$R_3$ = $C_{1-4}$-Alkyl;
$R_4$ = $C_{1-4}$-Alkyl bedeuten und die gestrichelte Linie eine gegebenenfalls vorhandene Doppelbindungen in der 1,2-Stellung angibt, indem man die entsprechende 21-OH-Verbindung in die entsprechende 21-Halogenverbindung, oder das entsprechende 9β, 11β-Epoxid in die entsprechende 9α-Halogen-, 11β-OH-Verbindung, oder die entsprechende 21-I-Verbindung in die entsprechende 21-Br-, 21-Cl- oder 21-F-Verbindung überführt, und die nach obiger Vorschrift erhaltene Verbindung gegebenenfalls mit einer Doppelbindung in $C_1$,$C_2$-Stellung versieht oder deren Doppelbindung in der $C_1$,$C_2$-Stellung hydriert und/oder ihre 11β-OH-Gruppe oxidiert.

**Revendications**

1. Nouveaux dérivés du prégnane répondant à la formule

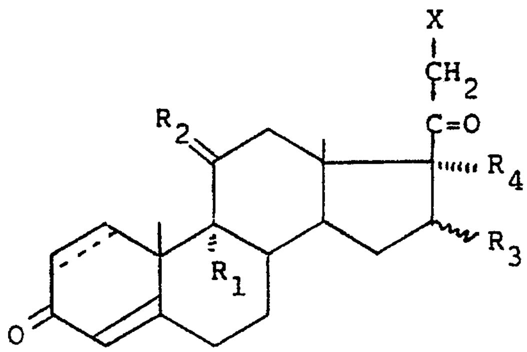

dans laquelle
X = F, Cl ou Br;
$R_1$ = F ou Cl;
$R_2$ = H(βOH) ou O;
$R_3$ = un groupe alkyle en $C_1$–$C_4$;
$R_4$ = un groupe alkyle en $C_1$–$C_4$; et la ligne en pointillé indique la présence facultative d'une double liaison en position 1,2.

2. Composé selon la revendication 1, caractérisé par le fait que X = Cl.
3. Composé selon la revendication 1 ou 2, caractérisé par le fait que $R_1$ = F.
4. Composé selon les revendications 1 à 3, caractérisé par le fait que $R_2$ = H(βOH).
5. Composé selon les revendications 1 à 4, caractérisé par le fait que $R_3$ = $CH_3$.
6. Composé selon les revendications 1 à 5, caractérisé par le fait que $R_3$ = α-$CH_3$.
7. Composé selon les revendications 1 à 6, caractérisé par le fait que $R_4$ = $CH_3$.
8. Composé selon les revendications 1 à 7, caractérisé par le fait que la double liaison en position 1,2 est présente.
9. Procédé pour préparer un dérivé du prégnane répondant à la formule:

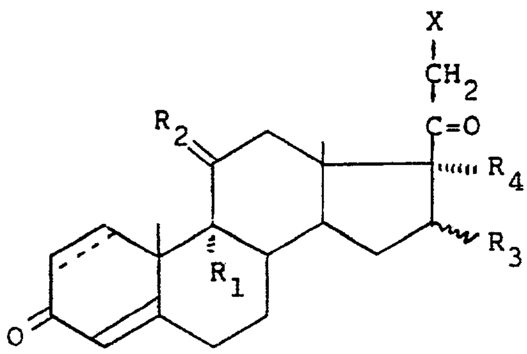

dans laquelle
X = F, Cl ou Br;
$R_1$ = F ou Cl;
$R_2$ = H(OH) ou O;
$R_3$ = un groupe alkyle en $C_1$–$C_4$;
$R_4$ = un groupe alkyle en $C_1$–$C_4$; et la ligne en pointillé indique la présence facultative d'une double liaison en position 1,2,

en transformant le composé 21-OH correspondant en le composé 21-halogéné correspondant, ou le 9β,11β-époxyde correspondant en le composé 9α-halogéno-11β-OH correspondant, ou le composé $\Delta^{9(11)}$ correspondant en le composé 9α-Cl-11β-OH corrrespondant, ou le composé 21-I correspondant en le composé 21-Br, 21-Cl ou 21-F correspondant et, facultativement, en dotant le composé obtenu selon les méthodes ci-dessus d'une double liaison en position 1,2 ou en hydrogénant sa double liaison en position 1,2 et/ou en oxydant son groupe 11β-OH.